# EUROPÄISCHE PATENTSCHRIFT

(11) **EP 2 755 586 B1**
(45) Veröffentlichungstag und Bekanntmachung des Hinweises auf die Patenterteilung: **02.03.2016**
(21) Anmeldenummer: 12761956.7
(22) Anmeldetag: 14.09.2012
(51) Int. Cl.: A61B 18/14

(54) **ELEKTROCHIRURGISCHES INSTRUMENT**
ELECTROSURGICAL INSTRUMENT
INSTRUMENT ÉLECTROCHIRURGICAL

(30) Priorität: 16.09.2011 DE 102011053682
(43) Veröffentlichungstag der Anmeldung: 23.07.2014
(73) Patentinhaber: Aesculap AG, 78532 Tuttlingen (DE)
(72) Erfinder: LUTZE, Theodor, 78582 Balgheim (DE); KELLER, Anton, 78589 Dürbheim (DE); WEISSHAUPT, Dieter, 78194 Immendingen (DE); EICK, Stefan, 78532 Tuttlingen (DE); ROTHWEILER, Christoph, 78166 Donaueschingen (DE); HERNER, Eugen, 72336 Balingen (DE)
(74) Vertreter: Winter, Brandl, Fürniss, Hübner, Röss, Kaiser, Polte - Partnerschaft mbB
(86) Internationale Anmeldenummer: PCT/EP2012/068158
(87) Internationale Veröffentlichungsnummer: WO 2013/037975

(56) Entgegenhaltungen:
- WO-A1-02/080796
- US-A1- 2004 162 557
- US-A1- 2011 184 404

## Beschreibung

Die Erfindung betrifft ein elektrochirurgisches Instrument mit einem proximalen Ende und einem distalen Ende, umfassend eine Maulteilanordnung am distalen Ende, die ein erstes Maulteil sowie ein relativ zu diesem bewegliches zweites Maulteil aufweist, wobei die Maulteile relativ zueinander von einer Öffnungsstellung in eine Greifstellung überführbar sind, in welcher Greifstellung sie näher aneinander angeordnet sind als in der Öffnungsstellung, wobei jedes Maulteil mindestens eine mit einer elektrischen Energiequelle verbindbare Elektrode aufweist und Elektroden beider Maulteile zum Versiegeln von in der Greifstellung zwischen den Maulteilen gehaltenem Körpergewebe zusammenwirken können, eine Betätigungseinrichtung zum Überführen der Maulteile von der Öffnungsstellung in die Greifstellung und eine Einrichtung zum Begrenzen der Schließweite der Maulteile, wenn diese von der Öffnungsstellung in die Greifstellung überführt werden.

Ein derartiges elektrochirurgisches Instrument ist beispielsweise in der EP 1 123 058 B1 und in der EP 1 372 507 B1 beschrieben. Um Körpergewebe mittels des Instrumentes zu versiegeln, können die Maulteile mittels der Betätigungseinrichtung von der Öffnungsstellung in die Greifstellung überführt werden, so dass zu versiegelndes Gewebe zwischen den Maulteilen gehalten ist. Die Greifstellung kann vorliegend auch als Schließstellung bezeichnet werden. Durch zusätzliches Anlegen einer elektrischen Spannung zwischen den Elektroden beider Maulteile kann das Körpergewebe elektrochirurgisch versiegelt werden. Die Einrichtung zur Begrenzung der Schließweite, nachfolgend auch vereinfacht nur "Einrichtung" genannt, ist vorgesehen, damit die Maulteile in der Greifstellung nicht so nahe aneinander angenähert sind, dass die mindestens eine Elektrode des ersten Maulteils und die mindestens eine Elektrode des zweiten Maulteils einander kontaktieren. Dadurch soll ein elektrischer Kurzschluss zwischen den Elektroden der Maulteile vermieden werden.

Aus US 2011/0184404 A1 ist elektrochirurgisches Instrument bekannt, bei welchem lediglich an einer Instrumentenbranche drei quer verlaufende und als Abstandshalter dienende Isolierstreifen vorgesehen sind. Dieses Dokument offenbart die Bezeichnung des Gegenstands des Anspruchs 1. Das Dokument US 2004/0162557 A1 offenbart ein elektrochirurgisches Instrument mit Abstandsgliedern. Die Erfindung wird im Anspruch 1 definiert und weitere Ausführungsformen finden sich in den abhängigen Ansprüchen.

"Proximal" und "distal" sind vorliegend auf einen das Instrument handhabenden Benutzer bezogen aufzufassen. Der Benutzer handhabt das Instrument vom proximalen Ende und wirkt mit dem distalen Ende auf Körpergewebe ein.

Aufgabe der vorliegenden Erfindung ist es, ein elektrochirurgisches Instrument der eingangs genannten Art bereitzustellen, mit dem ein verbessertes Versiegelungsergebnis erzielbar ist.

Diese Aufgabe wird bei einem gattungsgemäßen elektrochirurgischen Instrument erfingdungsgemäß dadurch gelöst, dass die Einrichtung eine Mehrzahl von Begrenzungselementen aufweist, von denen an einem Maulteil maximal zwei Begrenzungselemente angeordnet sind, die als vom Maulteil in Richtung des anderen Maulteils über eine von der mindestens einen Elektrode des Maulteils ausgebildete Versiegelungsfläche hervorspringendes, elektrisch isolierendes Abstandsglied ausgestaltet sind, wovon mindestens ein Abstandsglied an einem distalen Endbereich der Maulteilanordnung angeordnet ist.

Die Einrichtung zum Begrenzen der Schließweite umfasst eine Mehrzahl von Begrenzungselementen. Die Begrenzungselemente können als Abstandsglieder ausgestaltet sein, wobei zumindest ein Abstandsglied vorgesehen ist. Das Abstandsglied ragt über die Versiegelungsfläche der mindestens einen Elektrode desjenigen Maulteils, an dem es festgelegt ist, in Richtung des anderen Maulteils hinaus und kann das andere Maulteil kontaktieren. Mittels des mindestens einen Abstandsgliedes kann weitgehend vermieden werden, dass die Elektroden einander in der Greifstellung kontaktieren, so dass auch ein Kurzschluss weitgehend vermeidbar ist. Mindestens ein Abstandsglied ist an einem distalen Endbereich, insbesondere am distalen Ende der Maulteilanordnung angeordnet. Dadurch lässt sich die Schließweite der Maulteile am distalen Endbereich begrenzen, was in der Praxis insbesondere dann von Vorteil ist, wenn die Maulteile am distalen Ende aufeinander zu gekrümmt ausgebildet sind oder relativ zueinander vorgespannt sind. Weiter sind insgesamt an einem Maulteil maximal zwei Abstandsglieder vorgesehen, wobei an jedem der Maulteile 0, 1 oder 2 Abstandsglieder vorgesehen sein können. Dies erweist sich in der Praxis als ausreichend zum Vermeiden eines Kurzschlusses der Elektroden in der Greifstellung einerseits und als vorteilhaft für eine gute Versiegelung des Körpergewebes andererseits, weil die Anzahl der Abstandsglieder auf eine möglichst geringe Zahl beschränkt ist. Im Ergebnis erhält man mittels des erfindungsgemäßen Instrumentes ein verbessertes Versiegelungsergebnis.

Bei einer vorteilhaften Ausführungsform des erfindungsgemäßen Instrumentes erweist es sich als günstig, wenn insgesamt nur zwei als Abstandglieder ausgestaltete Begrenzungselemente vorgesehen sind. Hiervon ist mindestens eines, wie vorstehend erläutert, an einem distalen Endbereich der Maulteilanordnung angeordnet.

Die zwei Abstandsglieder können beispielsweise an verschiedenen Maulteilen angeordnet sein, so dass an jedem Maulteil ein Abstandsglied angeordnet ist.

Denkbar ist auch, dass die zwei Abstandsglieder an demselben Maulteil angeordnet sind und an dem jeweils anderen Maulteil kein Abstandsglied angeordnet ist.

Bei einer andersartigen vorteilhaften Ausführungsform kann vorgesehen sein, dass an jedem Maulteil zumindest ein Abstandsglied angeordnet ist, wovon zumindest eines am distalen Endbereich der Maulteilanordnung angeordnet ist.

Ferner kann vorgesehen sein, dass zwei, an einem Maulteil angeordnete Abstandsglieder in proximal-distaler Richtung an derselben oder im Wesentlichen an derselben Position angeordnet sind, wobei die zwei Abstandsglieder beispielsweise quer zur proximal-distalen Richtung seitlich nebeneinander am Maulteil angeordnet sein können. Dies erlaubt es beispielsweise, in der Greifstellung Querverspannungen zwischen den Maulteilen zu vermeiden, insbesondere wenn die Abstandsglieder symmetrisch relativ zueinander quer zur proximal-distalen Richtung am Maulteil angeordnet sind. Dadurch kann das Körpergewebe besser gefasst werden.

Es kann vorgesehen sein, dass mindestens zwei Abstandsgliedar, und insbesondere alle Abstandsglieder, identisch ausgestaltet sind.

Bei einer weiteren vorteilhaften Ausführungsform des erfindungsgemäßen Instrumentes kann vorgesehen sein, dass am distalen Endbereich der Maulteilanordnung genau ein Abstandsglied angeordnet ist.

Beispielsweise ist das Abstandsglied am distalen Endbereich mittig angeordnet, bezogen auf eine quer zur proximal-distalen Richtung ausgerichtete Richtung.

Weiter ist günstigerweise vorgesehen, dass zumindest ein oder auch genau ein Abstandsglied proximal bezüglich des distalen Endbereiches, und insbesondere an einem proximalen Endbereich des Maulteils angeordnet ist. Eine Begrenzung der Schließweite kann dadurch auch proximal, etwa am proximalen Endbereich des Maulteils erzielt werden und dementsprechend ein elektrischer Kurzschluss vermieden werden.

Es kann vorzugsweise vorgesehen sein, dass zumindest ein Abstandsglied in die mindestens eine Elektrode desjenigen Maulteils integriert ist, an dem es angeordnet ist. Beispielsweise kann die Elektrode eine Ausnehmung umfassen, in die das Abstandsglied eingesetzt ist. Die Elektrode kann auch eine Durchbrechung aufweisen, durch die hindurch das Abstandsglied die Elektrode in Richtung des jeweils anderen Maulteils durchsetzt.

Insbesondere können alle Abstandsglieder in die mindestens eine Elektrode desjenigen Maulteils integriert sein, an dem sie festgelegt sind.

Günstig ist es auch, wenn mindestens ein Abstandsglied einstückig mit einem Trägerelement desjenigen Maulteils, an dem es angeordnet ist, für die mindestens eine Elektrode dieses Maulteils gebildet ist. Dadurch kann eine konstruktiv einfache Ausgestaltung des Instrumentes erzielt werden.

Insbesondere können alle Abstandsglieder einstückig mit einem Trägerelement desjenigen Maulteils, an dem sie jeweils angeordnet sind, für die mindestens eine Elektrode dieses Maulteils gebildet sein.

Von Vorteil ist es, wenn das Instrument eine Schneideinrichtung umfasst zum Durchtrennen von zwischen den Maulteilen in deren Greifstellung gehaltenem Körpergewebe, wobei die Schneideinrichtung ein Schneidelement aufweist, das quer zur proximal-distalen Richtung zwischen zwei Versiegelungsflächenabschnitten der jeweiligen mindestens einen Elektrode der Maulteile angeordnet oder anordenbar ist. Mittels eines derartigen Instrumentes kann das Gewebe nicht nur versiegelt werden, sondern auch mittels des Schneidelementes durchtrennt werden. Quer zur proximal-distalen Richtung befinden sich an jedem Maulteil zwei Versiegelungsflächenabschnitte, so dass das Körpergewebe beiderseits des Schneidelementes versiegelt werden kann.

Bei einer vorteilhaften Ausführungsform des Instrumentes kann vorgesehen sein, dass das Schneidelement an einem der Maulteile gehalten ist.

Bei einer andersartigen vorteilhaften Ausführungsform des Instrument umfasst die Schneideinrichtung ein Betätigungselement zum Bewegen des Schneidelementes in proximal-distaler Richtung und zu dessen Überführen von einer zurückgezogenen Stellung in eine vorgeschobene Stellung, wobei beim Überführen zwischen den Maulteilen in deren Greifstellung gehaltenes Körpergewebe durchtrennt wird. Dies erlaubt einem Benutzer, die Schneidfunktion des Instrumentes nur bei Bedarf durch Einwirken auf das Betätigungselement zu aktivieren.

Denkbar ist es, dass zumindest ein Maulteil zwei getrennte Elektroden aufweist bzw. aufweisen, von denen jede einen Versiegelungsflächenabschnitt ausbildet.

Weiterhin ist vorstellbar, dass zumindest ein Maulteil eine Elektrode aufweist, die beide Versiegelungsflächenabschnitte ausbildet.

In der Praxis erweist es sich als vorteilhaft für ein gleichmäßiges Begrenzen der Schließweite, wenn quer zur proximal-distalen Richtung zumindest ein Abstandsglied zu jeder Seite des Schneidelementes angeordnet ist. Im Falle des vorstehend erwähnten beweglichen Schneidelementes ist dies auf eine Position desselben bezogen aufzufassen, die es beim Überführen von der zurückgezogenen in die vorgeschobene Stellung zum Durchtrennen von Gewebe zwischen den Versiegelungsflächenabschnitten einnehmen kann.

Insbesondere kann vorgesehen sein, dass genau ein Abstandsglied zu jeder Seite des Schneidelementes angeordnet ist.

Als günstig erweist es sich, wenn die Einrichtung zum Begrenzen der Schließweite mindestens ein weiteres, nicht als Abstandsglied ausgestaltetes Begrenzungselement umfasst, das proximal bezüglich des mindestens einen, am distalen Endbereich der Maulteilanordnung angeordneten Abstandsgliedes angeordnet ist. Dies gibt die Möglichkeit, die Schließweite der Maulteilen noch zuverlässiger zu begrenzen, und zwar zum Einen mittels des mindestens einen Abstandsgliedes am distalen Endbereich und zum anderen mittels eines weiteren, proximaleren Begrenzungselementes.

Von Vorteil ist es, wenn das Instrument einen Maulteilträger für das erste Maulteil aufweist, relativ zu dem das zweite Maulteil verschwenkbar ist zum Überführen der Maulteile von der Öffnungsstellung in die Greifstellung, und wenn das erste Maulteil relativ zum Maulteilträger um eine quer zu einer von diesem definierten Längsrichtung orientierte Schwenkachse schwenkbar ist. Relativ zum Maulteilträger kann das erste Maulteil verschwenkt werden. Dies ist von Vorteil, da beim Überführen der Maulteile in die Greifstellung sich deren Elektroden parallel zueinander aneinander annähern können, so dass die Maulteile idealerweise in proximal-distaler Richtung und quer dazu einen gleichmäßigen Abstand über die gesamten Versiegelungsflächen der Elektroden aufweisen können. Das Körpergewebe kann dadurch gleichmäßig und zuverlässig versiegelt werden.

Es kann vorgesehen sein, dass das distale Ende des ersten Maulteils in Richtung auf das distale Ende des zweiten Maulteils vorgespannt ist, um zu versiegelndes Gewebe zu hintergreifen und damit besser zu fassen.

Günstig ist es, wenn die Einrichtung zum Begrenzen der Schließweite mindestens ein weiteres, als erstes Anschlagsglied ausgestaltetes Begrenzungselement umfasst, das am ersten Mauiteil angeordnet ist, sowie mindestens ein weiteres, als zweites Anschlagsglied ausgestaltetes Begrenzungselement, das am Maulteilträger angeordnet ist und das zum Begrenzen der Schwenkbewegung des ersten Maulteils relativ zum Maulteilträger derart, dass das proximale Ende des ersten Maulteils zum zweiten Maulteil hin verschwenkt wird, zusammenwirkt. Durch die weiteren, als Anschlagsglieder ausgestalteten Begrenzungselemente kann die Schließweite der Maulteilen noch zuverlässiger begrenzt werden.

Es kann insbesondere vorgesehen sein, dass die Anschlagsglieder die Schwenkbewegung derart begrenzen, dass bei deren Zusammenwirken die von der mindestens einen Elektrode des ersten Maulteiles ausgebildete Versiegelungsfläche parallel zur vom Maulteilträger definierten Längsrichtung ausgerichtet ist.

Bei einer konstruktiv einfachen Ausgestaltung kann vorgesehen sein, dass die Anschlagsglieder als aneinander anlegbare Absätze des ersten Maulteils bzw. des Maulteilträger ausgebildet sind.

Bevorzugt bilden die Anschlagsglieder Führungsglieder zum Führen des ersten Maulteils beim Verschwenken relativ zum Maulteilträger aus. Dadurch kann das Maulteil in definierter Weise verschwenkt und die Schließseite der Maulteile zusätzlich begrenzt werden.

Alternativ oder ergänzend kann vorgesehen sein, dass ein Anschlagglied als Langloch ausgebildet ist, das mit ihm zusammenwirkende Anschlagsglied als in das Langloch eingreifender Zapfen. Derartige Anschlagsglieder können beispielsweise die vorstehend erwähnten Führungsglieder ausbilden.

Alternativ oder ergänzend kann vorgesehen sein, dass das mindestens eine Anschlagsglied ein verstellbares Stellglied ausbildet, das relativ zum ersten Maulteil bzw. relativ zum Maulteilträger verstellbar ist. Dies gibt die Möglichkeit, die Schließseite durch eine Manipulation des Stellglieds einzustellen, beispielsweise werksseitig oder anwenderseitig.

Günstig ist es, wenn das Stellglied feststellbar verstellbar ist.

Bei einer vorteilhaften Ausführungsform des Instrumentes kann vorgesehen sein, dass die Anschlagsglieder an einer dem zweiten Maulteil abgewandten Seite des ersten Maulteils und des Maulteilträger angeordnet sind.

Alternativ oder ergänzend kann vorgesehen sein, dass die Anschlagsglieder auf einer dem zweiten Maulteil zugewandten Seite des ersten Maulteils und des Maulteilträger angeordnet sind.

Zur Erzielung einer konstruktiv einfachen Ausgestaltung ist es günstig, wenn die Anschlagsglieder einstückig mit einem Trägerelement für die mindestens eine Elektrode des ersten Maulteils bzw. einstückig mit dem Maulteilträger gebildet sind.

Eine vorteilhafte Ausführungsform des erfindungsgemäßen Instrumentes weist einen Schaft auf, an dessen distalem Ende die Maulteilanordnung angeordnet ist, wobei zumindest ein Maulteil relativ zum Schaft verschwenkbar ist. Der Schaft ist beispielsweise der vorstehend erwähnte Maulteilträger, relativ zu dem das zweite Maulteil verschwenkbar ist. Es kann auch vorgesehen sein, dass beide Maulteile relativ zum Schaft verschwenkbar sind.

Bei einer andersartigen vorteilhaften Ausführungsform ist es günstig, wenn das Instrument zwei relativ zueinander verschwenkbare Maulteilträger umfasst, wobei an einem jeweiligen distalen Ende jedes Maulteilträgers eines der beiden Maulteile angeordnet ist. Die Maulteilträger bei dieser Ausführungsform bilden beispielsweise Branchen, die um eine quer zur proximal-distalen Richtung ausgerichtete Schwenkachse verschwenkbar sind.

Die vorstehende Beschreibung umfasst somit insbesondere die nachfolgend in Form durchnummerierter Sätze definierten Ausführungsformen eines elektrochirurgischen Instruments:
1. Elektrochirurgisches Instrument mit einem proximalen Ende und einem distalen Ende, umfassend:
   - eine Maulteilanordnung am distalen Ende, die ein erstes Maulteil sowie ein relativ zu diesem bewegliches zweites Maulteil aufweist, wobei die Maulteilen relativ zueinander von einer Öffnungsstellung in eine Greifstellung überführbar sind, in welcher Greifstellung sie näher aneinander angeordnet sind als in der Öffnungsstellung, wobei jedes Maulteil mindestens eine mit einer elektrischen Energiequelle verbindbare Elektrode aufweist und Elektroden beider Maulteile zum Versiegeln von in der Greifstellung zwischen den Maulteilen gehaltenem Körpergewebe zusammenwirken können;
   - eine Betätigungseinrichtung zum Überführen der Maulteile von der Öffnungsstellung in die Greifstellung; und
   - eine Einrichtung zum Begrenzen der Schließweite der Maulteile, wenn diese von der Öffnungsstellung in die Greifstellung überführt werden,
      **dadurch gekennzeichnet**, dass die Einrichtung eine Mehrzahl von Begrenzungselementen aufweist, von denen an einem Maulteil maximal zwei Begrenzungselemente angeordnet sind, die als vom Maulteil in Richtung des anderen Maulteils über eine von der mindestens einen Elektrode des Maulteils ausgebildete Versiegelungsfläche hervorspringendes, elektrisch isolierendes Abstandsglied ausgestaltet sind, wovon mindestens ein Abstandsglied an einem distalen Endbereich der Maulteilanordnung angeordnet ist.
2. Instrument nach Satz 1, dadurch gekennzeichnet, dass insgesamt nur zwei als Abstandglieder ausgestaltetes Begrenzungselemente vorgesehen sind.
3. Instrument nach Satz 2, dadurch gekennzeichnet, dass die zwei Abstandsglieder an verschiedenen Maulteilen angeordnet sind.
4. Instrument nach Satz 2, dadurch gekennzeichnet, dass die zwei Abstandsglieder an demselben Maulteil angeordnet sind.
5. Instrument nach einem der voranstehenden Sätze, dadurch gekennzeichnet, dass an jedem Maulteil zumindest ein Abstandsglied angeordnet ist.
6. Instrument nach einem der voranstehenden Sätze, dadurch gekennzeichnet, dass zwei, an einem Maulteil angeordnete Abstandsglieder in proximal-distaler Richtung an derselben oder im Wesentlichen an derselben Position angeordnet sind.
7. Instrument nach Satz 6, dadurch gekennzeichnet, dass die zwei Abstandsglieder quer zur proximal-distalen Richtung seitlich nebeneinander am Maulteil angeordnet sind.
8. Instrument nach einem der voranstehenden Sätze, dadurch gekennzeichnet, dass am distalen Endbereich der Maulteilanordnung genau ein Abstandsglied angeordnet ist.
9. Instrument nach Satz 8, dadurch gekennzeichnet, dass das Abstandsglied am distalen Endbereich mittig angeordnet ist.
10. Instrument nach einem der voranstehenden Sätze, dadurch gekennzeichnet, dass zumindest ein Abstandsglied proximal bezüglich des distalen Endbereiches, insbesondere an einem proximalen Endbereich des Maulteils angeordnet ist.
11. Instrument nach einem der voranstehenden Sätze, dadurch gekennzeichnet, dass zumindest ein Abstandsglied in die mindestens eine Elektrode desjenigen Maulteils integriert ist, an dem es angeordnet ist.
12. Instrument nach Satz 11, dadurch gekennzeichnet, dass alle Abstandsglieder in die mindestens eine Elektrode desjenigen Maulteils integriert sind, an dem sie festgelegt sind.
13. Instrument nach einem der voranstehenden Sätze, dadurch gekennzeichnet, dass mindestens ein Abstandsglied einstückig mit einem Trägerelement desjenigen Maulteils, an dem es angeordnet ist, für die mindestens eine Elektrode dieses Maulteils gebildet ist.
14. Instrument nach Satz 13, dadurch gekennzeichnet, dass alle Abstandsglieder einstückig mit einem Trägerelement desjenigen Maulteils, an dem sie jeweils angeordnet sind, für die mindestens eine Elektrode dieses Maulteils gebildet sind.
15. Instrument nach einem der voranstehenden Sätze, dadurch gekennzeichnet, dass das Instrument eine Schneideinrichtung umfasst zum Durchtrennen von zwischen den Maulteilen in deren Greifstellung gehaltenem Körpergewebe, wobei die Schneideinrichtung ein Schneidelement aufweist, das quer zur proximal-distalen Richtung zwischen zwei Versiegelungsflächenabschnitten der jeweiligen mindestens einen Elektroden der Maulteilen angeordnet oder anordenbar ist.
16. Instrument nach Satz 15, dadurch gekennzeichnet, dass das Schneidelement an einem der Maulteile gehalten ist.
17. Instrument nach Satz 15, dadurch gekennzeichnet, dass die Schneideinrichtung ein Betätigungselement umfasst zum Bewegen des Schneidelementes in proximal-distaler Richtung und zu dessen Überführen von einer zurückgezogenen Stellung in eine vorgeschobene Stellung, wobei beim Überführen zwischen den Maulteilen in deren Greifstellung gehaltenes Körpergewebe durchtrennt wird.
18. Instrument nach einem der Sätze 15 bis 17, dadurch gekennzeichnet, dass zumindest ein Maulteil zwei getrennte Elektroden aufweist, von denen jede einen Versiegelungsflächenabschnitt ausbildet.
19. Instrument nach einem der Sätze 15 bis 18, dadurch gekennzeichnet, dass zumindest ein Maulteil eine Elektrode aufweist, die beide Versiegelungsflächenabschnitte ausbildet.
20. Instrument nach einem der Sätze 15 bis 19, dadurch gekennzeichnet, dass quer zur proximal-distalen Richtung zumindest ein Abstandsglied zu jeder Seite des Schneidelementes angeordnet ist.
21. Instrument nach Satz 20, dadurch gekennzeichnet, dass genau ein Abstandsglied zu jeder Seite des Schneidelementes angeordnet ist.
22. Instrument nach einem der voranstehenden Sätze, dadurch gekennzeichnet, dass die Einrichtung zum Begrenzen der Schließweite mindestens ein weiteres, nicht als Abstandsglied ausgestaltetes Begrenzungselement umfasst, das proximal bezüglich des mindestens einen, am distalen Endbereich der Maulteilanordnung angeordneten Abstandsgliedes angeordnet ist.
23. Instrument nach einem der voranstehenden Sätze, dadurch gekennzeichnet, dass das Instrument einen Maulteilträger für das erste Maulteil aufweist, relativ zu dem das zweite Maulteil verschwenkbar ist zum Überführen der Maulteile von der Öffnungsstellung in die Greifstellung, und dass das erste Maulteil- relativ zum Maulteilträger um eine quer zu einer von diesem definierten Längsrichtung ausgerichtete Schwenkachse schwenkbar ist.
24. Instrument nach Satz 23, dadurch gekennzeichnet, dass die Einrichtung zum Begrenzen der Schließweite mindestens ein weiteres, als erstes Anschlagsglied ausgestaltetes Begrenzungselement umfasst, das am ersten Maulteils angeordnet ist, sowie mindestens ein weiteres, als zweites Anschlagsglied ausgestaltetes Begrenzungselement, das am Maulteilträger angeordnet ist und das zum Begrenzen der Schwenkbewegung des ersten Maulteils relativ zum Maulteilträger derart, dass das proximale Ende des ersten Maulteils hin zum zweiten Maulteil verschwenkt wird, zusammenwirkt.
25. Instrument nach Satz 24, dadurch gekennzeichnet, dass die Anschlagsglieder die Schwenkbewegung derart begrenzen, dass bei deren Zusammenwirken die von der mindestens einen Elektrode des ersten Maulteils ausgebildete Versiegelungsfläche parallel zur vom Maulteilträger definierten Längsrichtung ausgerichtet ist.
26. Instrument nach Satz 24 oder 25, dadurch gekennzeichnet, dass die Anschlagsglieder als aneinander anlegbare Absätze des ersten Maulteils bzw. des Maulteilträger ausgebildet sind.
27. Instrument nach einem der Sätze 24 bis 26, dadurch gekennzeichnet, dass die Anschlagsglieder Führungsglieder zum Führen des ersten Maulteils beim Verschwenken relativ zum Maulteilträger ausbilden.
28. Instrument nach einem der Sätze 24 bis 27, dadurch gekennzeichnet, dass ein Anschlagsglied als Langloch ausgebildet ist, das mit ihm zusammenwirkende Anschlagsglied als in das Langloch eingreifender Zapfen.
29. Instrument nach einem der Sätze 24 bis 28, dadurch gekennzeichnet, dass mindestens ein Anschlagsglied ein verstellbares Stellglied ausbildet, das relativ zum ersten Maulteil bzw, relativ zum Maulteilträger verstellbar ist.
30. Instrument nach Satz 29, dadurch gekennzeichnet, dass das Stellglied feststellbar verstellbar ist.
31. Instrument nach einem der Sätze 24 bis 30, dadurch gekennzeichnet, dass die Anschlagsglieder an einer dem zweiten Maulteil abgewandten Seite des ersten Maulteils und des Maulteilträger angeordnet sind.
32. Instrument nach einem der Sätze 24 bis 31, dadurch gekennzeichnet, dass die Anschlagsglieder an einer dem zweiten Maulteil zugewandten Seite des ersten Maulteils und des Maulteilträgers angeordnet sind.
33. Instrument nach einem der Sätze 24 bis 32, dadurch gekennzeichnet, dass die Anschlagsglieder einstückig mit einem Trägerelement für die mindestens eine Elektrode des ersten Maulteils bzw. einstückig mit dem Maulteilträger gebildet sind.
34. Instrument nach einem der voranstehenden Sätze, dadurch gekennzeichnet, dass das Instrument einen Schaft aufweist, an dessen distalem Ende die Maulteilanordnung angeordnet ist, wobei zumindest ein Maulteil relativ zum Schaft verschwenkbar ist.
35. Instrument nach einem der Sätze 1 bis 33, dadurch gekennzeichnet, dass das Instrument zwei relativ zueinander verschwenkbare Maulteilträger umfasst, wobei an einem jeweiligen distalen Ende jedes Maulteilträgers eines der beiden Maulteile angeordnet ist.

Die nachfolgende Beschreibung bevorzugter Ausführungsformen der Erfindung dient im Zusammenhang mit der Zeichnung der näheren Erläuterung der Erfindung. Es zeigen:
- Figur 1:: eine perspektivische Darstellung einer ersten bevorzugten Ausführungsform eines erfindungsgemäßen elektrochirurgischen Instrumentes, das einen Schaft und an dessen distalem Ende eine Maulteilanordnung mit zwei relativ zueinander beweglichen Maulteilen aufweist, wobei die Mauiteile in einer Öffnungsstellung dargestellt sind;
- Figur 2:: eine vergrößerte Darstellung von Detail "2" in Figur 1;
- Figur 3:: eine Draufsicht auf ein distales Ende des Schaftes und ein erstes Maulteil des Instrumentes aus Figur 1;
- Figur 4:: eine Draufsicht auf das andere Maulteil des Instrumentes aus Figur 1;
- Figur 5:: eine stirnseitige Ansicht des Instruments aus Figur 1 in distalproximaler Richtung, wobei die Maulteile eine Greifstellung einnehmen;
- Figur 6A:: eine Ansicht entsprechend Figur 2 bei einer zweiten bevorzugten Ausführungsform des erfindungsgemäßen Instrumentes;
- Figur 6B:: eine Draufsicht auf ein distales Ende des Schaftes und ein Maulteil der zweiten bevorzugten Ausführungsform des Instrumentes;
- Figur 7A:: eine Seitenansicht des distalen Endes des Schaftes und der Maulteilanordnung aus Figur 6, teilweise geschnitten, wobei die Maulteile die Öffnungsstellung einnehmen;
- Figur 7B:: eine Darstellung von Detail A in Figur 7A;
- Figur 8A:: eine Darstellung entsprechend Figur 7A, wobei die Maulteile die Greifstellung einnehmen;
- Figur 8B:: eine Darstellung von Detail B in Figur 8A;
- Figur 9A:: eine Darstellung entsprechend Figur 7A bei einer dritten bevorzugten Ausführungsform des erfindungsgemäßen Instrumentes, wobei die Maulteilen eine Öffnungsstellung einnehmen;
- Figur 9B:: eine Darstellung von Detail C in Figur 9A;
- Figur 10A:: eine Darstellung entsprechend Figur 9A, wobei die Maulteile eine Greifstellung einnehmen;
- Figur 10B:: eine Darstellung von Detail D in Figur 10A;
- Figur 11A:: eine Darstellung entsprechend Figur 7A bei einer vierten bevorzugten Ausführungsform des erfindungsgemäßen Instrumentes, wobei die Maulteile eine Öffnungsstellung einnehmen;
- Figur 11B:: eine Darstellung von Detail E in Figur 11A;
- Figur 12A:: eine Darstellung entsprechend Figur 11A, wobei die Maulteile die Greifstellung einnehmen;
- Figur 12B:: eine Darstellung von Detail F in Figur 12A;
- Figur 13A:: eine Darstellung entsprechend Figur 2 bei einer fünften bevorzugten Ausführungsform des erfindungsgemäßen Instrumentes, wobei die Maulteilen eine Öffnungsstellung einnehmen und ein erstes Maulteil von am Schaft angeordneten Anschlagsgliedern beabstandet ist;
- Figur 13B:: eine Darstellung entsprechend Figur 13, wobei das erste Maulteil an den Anschlagsgliedern am Schaft anschlägt;
- Figur 14:: eine Ansicht entsprechend Figur 2 bei einer sechsten bevorzugten Ausführungsform des erfindungsgemäßen Instrumentes, wobei die Maulteile eine Öffnungsstellung einnehmen;
- Figur 15:: eine stirnseitige Ansicht des Instrumentes der sechsten Ausführungsform in distal-proximaler Richtung, wobei die Maulteile eine Greifstellung einnehmen;
- Figur 16:: eine Darstellung entsprechend Figur 2 bei einer siebten bevorzugten Ausführungsform des erfindungsgemäßen Instrumentes, wobei die Maulteilen eine Öffnungsstellung einnehmen;
- Figur 17A:: eine Seitenansicht der Ausführungsform gemäß Figur 16, wobei die Maulteile eine Greifstellung einnehmen;
- Figur 17B:: eine vergrößerte Darstellung von Detail G in Figur 17A und
- Figur 18:: eine perspektivische Darstellung einer achten bevorzugten Ausführungsform eines erfindungsgemäßen Instrumentes.

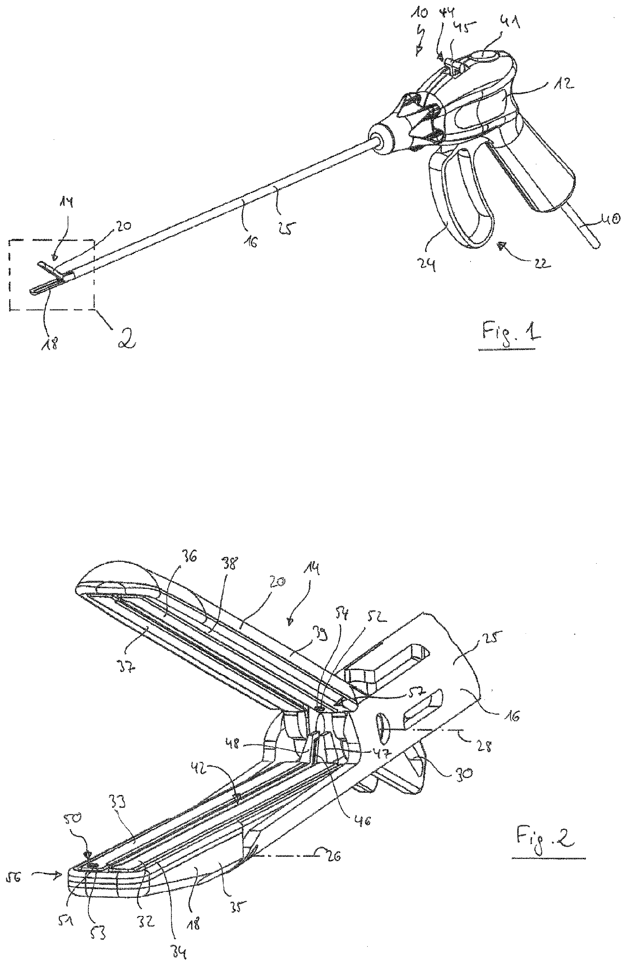

Figur 1 zeigt in perspektivischer Darstellung eine mit dem Bezugszeichen 10 belegte erste bevorzugte Ausführungsform eines erfindungsgemäßen Instrumentes zum Versiegeln und Schneiden von Körpergewebe. Das Instrument 10 ist als bipolares Rohrschaftinstrument ausgestaltet, das an einem proximalen Ende eine Handhabe 12 und an einem distalen Ende eine Maulteilanordnung 14 aufweist.

"Proximal" und "distal" sind vorliegend auf einen das Instrument 10 handhabenden Benutzer bezogen aufzufassen. Der Benutzer handhabt das Instrument 10 am proximalen Ende mit der Handhabe 12 und wirkt mit dem distalen Ende mit der Maulteilanordnung 14 auf Körpergewebe ein.

Die Maulteilanordnung 14 ist an einem geraden Schaft 16 gehalten, der in der Handhabe 12 verankert ist. In der Handhabe 12 und im Schaft 16 ist eine an sich bekannte Mechanik angeordnet, um zwei Maulteile 18 und 20 der Maulteilanordnung 14 relativ zueinander zu bewegen. Zu diesem Zweck weist das Instrument 10 ferner eine Betätigungseinrichtung 22 auf, die an der Handhabe 12 einen vom Benutzer betätigbaren Betätigungshebel 24 umfasst. Durch Zug am Betätigungshebel 24 können die Maulteile 18 und 20 in an sich bekannter Weise von einer Öffnungsstellung, die in den Figuren 1 und 2 dargestellt ist, in eine Greifstellung überführt werden.

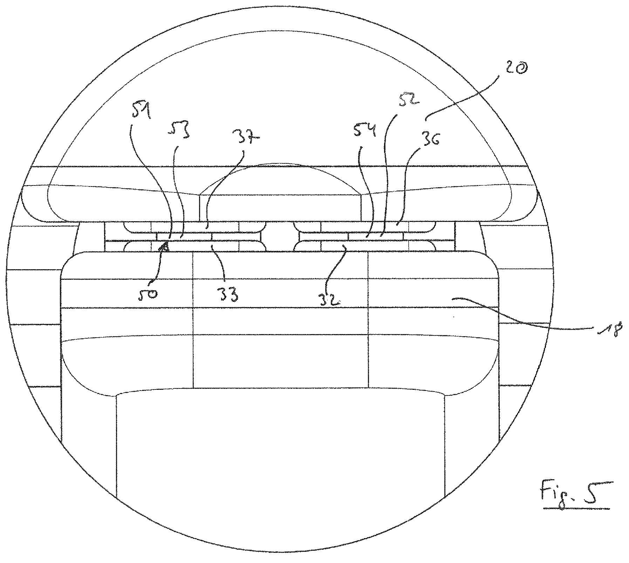

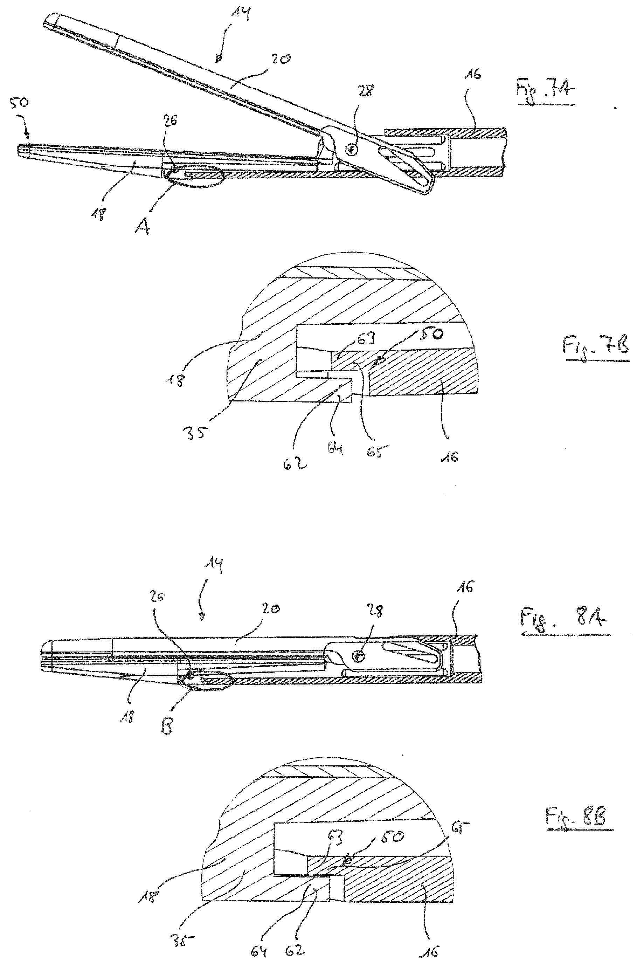

In der Öffnungsstellung sind die Maulteilen 18 und 20 relativ zueinander beabstandet, worunter vorliegend auch eine in den Figuren 1 und 2 dargestellte Spreizstellung der Maulteile 18 und 20 zu verstehen ist. Die Greifstellung der Maulteile 18 und 20 ist nur in der stirnseitigen Ansicht der Figur 5 dargestellt. In Seitenansicht nehmen die Maulteile 18 und 20 in der Greifstellung dann eine Relativposition zueinander ein, wie sie für den Fall der weiteren vorteilhaften Ausführungsformen des Instrumentes in den Figuren 8A, 10A, 12A und 17A dargestellt ist. In der Greifstellung sind die Maulteile 18 und 20 weniger weit voneinander beabstandet als in der Öffnungsstellung, worauf nachfolgend noch eingegangen wird.

Der Schaft 16 bildet einen Maulteilträger 25, an dessen distalem Ende das erste Maulteil 18 um eine quer zur vom Schaft 16 definierten Längsrichtung ausgerichtete Schwenkachse 26 schwenkbar gelagert ist. Das erste Maulteil 18 kann relativ zum Schaft 16 um einen vergleichsweise kleinen Winkeibereich schwenken, und sein distales Ende ist in Richtung auf das zweite Maulteil 20 durch eine in der Zeichnung nicht dargestellte Vorspanneinrichtung, etwa ein Federelement, vorgespannt.

Das zweite Maulteil 20 ist am Schaft 16 proximal bezüglich des ersten Mauiteils 18 um eine quer zur Längsrichtung des Schaftes 16 ausgerichtete Schwenkachse 28 schwenkbar gelagert. Ein im Schaft 16 in proximal-distaler Richtung geführtes und in der Zeichnung nicht dargestelltes Zugelement kann in eine Steuerkulisse 30 des zweiten Maulteils 20 eingreifen, so dass dieses, wie erwähnt, durch Einwirken auf den Betätigungshebel 24 von der Öffnungsstellung in die Greifstellung überführt wird. Dabei verschwenkt das Maulteil 20 um die Schwenkachse 28 in Richtung auf das Maulteil 18.

Das Maulteil 18 umfasst zum Versiegeln von Körpergewebe zwei quer zur proximal-distalen Richtung voneinander beabstandete, proximal-distal längserstreckte Elektroden 32 und 33. Die Elektroden 32 und 33 sind jeweils in einem elektrisch isolierenden Trägerelement 34 des Maulteils 18 gefasst, das seinerseits in einer Fassung 35 angeordnet ist, mittels derer das Maulteil 18 am Schaft 16 festgelegt ist.

In ähnlicher Weise weist das Maulteil 20 zwei quer zur Längsrichtung des Schaftes voneinander beabstandete, proximal-distal iängserstreckte Elektroden 36 und 37 auf, die ihrerseits in einem elektrisch isolierenden Trägerelement 38 gefasst sind. Das Trägerelement ist seinerseits in einer Fassung 39 angeordnet, mit der das Maulteil 20 am Schaft 16 festgelegt ist.

Die Elektroden 32, 33, 36 und 37 stehen über ein Anschlusskabel 40 des Instrumentes 10 mit einem in der Zeichnung nicht dargestellten elektrochirurgischen bipolaren Hochfrequenzgenerator in Verbindung und können von diesem unter Spannung gesetzt werden. Zu diesem Zweck kann ein Benutzer ein Betätigungsglied 41 an der Handhabe 12 betätigen.

Die Elektroden 32 und 33 liegen üblicherweise auf demselben Potential, ebenso die Elektroden 36 und 37, deren Potential sich von demjenigen der Elektroden 32 und 33 unterscheidet. In der Greifstellung der Maulteile 18 und 20 kann zwischen diesen gehaltenes Körpergewebe elektrochirurgisch versiegelt werden, indem zwischen den Elektroden 32 und 36 sowie zwischen den Elektroden 33 und 37 Strom fließt.

Anstelle von jeweils zwei Elektroden pro Maulteil könnte auch nur eine Elektrode pro Maulteil vorgesehen sein, oder auch eine größere Anzahl von Elektroden pro Maulteil.

Wie erwähnt, sind die Elektroden 32 und 33 sowie die Elektroden 36 und 37 quer zur Längsrichtung des Instrumentes 10 voneinander beabstandet. Ferner weisen die Trägerelemente 34 und 38 mittige Ausnehmungen auf, so dass zwischen den Elektroden 32 und 33 sowie 36 und 37 ein proximal-distaler Kanal 42 gebildet ist.

Das Instrument 10 umfasst eine Schneideinrichtung 44, die ein an der Handhabe 12 angeordnetes Betätigungsglied 45 umfasst sowie ein Schneidelement 46, auf das mit dem Betätigungsglied 45 eingewirkt werden kann. Das Schneidelement 46 ist in proximal-distaler Richtung verschieblich am Schaft 16 gelagert und kann ausgehend von einer zurückgezogenen Stellung (Figur 2) in eine in der Zeichnung nicht dargestellte vorgeschobene Stellung überführt werden.

In der zurückgezogenen Stellung ist das Schneidelement 46 zwischen pyramidenstumpfartigen Vorsprüngen 47 und 48 des Trägerelementes 34 an dessen proximalem Ende angeordnet. Von der zurückgezogenen Stellung kann das Schneidelement 46 erst nach dem Überführen der Maulteilen 18 und 20 in die Greifstellung in die vorgeschobene Stellung verschoben werden, wobei es im Kanal 42 bewegt wird. Dies erlaubt es, mit den Maulteilen 18 und 20 gefasstes und mit den Elektroden 32, 33, 36 und 37 versiegeltes Körpergewebe mittels des Schneidelementes 46 zu durchtrennen.

Das Instrument 10 weist, um eine zuverlässiges Versiegelung von Körpergewebe zu erzielen, eine Einrichtung zum Begrenzen der Schließseite der Maulteile 18 und 20 auf, nachfolgend vereinfacht als "einrichtung" 50 bezeichnet. Die Einrichtung 50 dient zum Begrenzen der Schließweite der Maulteile 18 und 20 relativ zueinander, wenn diese in die Greifstellung überführt werden. Dadurch kann weitgehend ein elektrischer Kurzschluss zwischen den Elektroden 32 und 36 sowie 33 und 37 vermieden werden. Das zu verspiegelnde Körpergewebe kann dadurch kontrollierbarer manipuliert und zuverlässiger versiegelt werden.

Die Einrichtung 50 umfasst zwei Begrenzungselemente 51 und 52 in Gestalt von Abstandsgliedern 53 bzw. 54. Die Abstandsglieder 53 und 54 sind elektrisch isolierend. Das Abstandsglied 53 ist in die Elektrode 33 integriert, und zwar an einem distalen Endbereich 56 der Maulteilanordnung 14. Beispielsweise ist das Abstandsglied 53 in einer Ausnehmung in der Elektrode angeordnet. Denkbar ist auch, dass das Abstandsglied 53 einstückig mit dem Trägerelement 34 gebildet ist und die Elektrode 33 durchgreift.

Das Abstandsglied 54 ist an einem proximalen Endbereich 57 der Maulteilanordnung 14 angeordnet und in die Elektrode 36 integriert. Damit umfasst die Einrichtung 50 zwei auf einander gegenüberliegenden Seiten des Kanals 42 sowie des daran anordenbaren Schneidelementes 46 angeordnete Abstandsglieder 53, 54, wovon zumindest eines im distalen Endbereich 56 angeordnet ist.

Die Abstandsglieder 53, 54 stehen über die von den Elektroden 32 und 33 einerseits bzw. 36 und 37 ausgebildeten elektrochirurgischen Versiegelungsflächen in Richtung des jeweils anderen Maulteils 20 bzw. 18 hinaus. Beispielsweise überragen die Abstandsglieder 53 und 54 die Elektroden 32, 33 bzw. 36, 37 um ungefähr 25 µm bis ungefähr 250 µm, bevorzugter um etwa 100 µm bis etwa 200 µm.

Wie erwähnt, kann mittels der Einrichtung 50 und damit mittels der Abstandsglieder 53 und 54 ein Kurzschluss der Elektroden 32 und 36 sowie 33 und 37 weitgehend verhindert werden. Werden die Maulteile 18 und 20 in die Greifstellung überfährt, um Körpergewebe zwischen ihnen zu halten, gelangt das Abstandsglied 53 an der Elektrode 37 zur Anlage, und das Abstandsglied 54 gelangt an der Elektrode 32 zur Anlage (Figur 5). Hierbei erfolgt zunächst die Kontaktierung der Elektrode 37 mittels des Abstandsglieds 53, da das Maulteil 18 distal in Richtung auf das Maulteil 20 vorgespannt ist.

Im Ergebnis kann mittels des Instruments 10 ein verbessertes Versiegelungsergebnis dadurch erzielt werden, dass ein Kurzschluss zwischen den Elektroden der Maulteile 18 und 20 selbst dann verhinderbar ist, wenn zu versiegelndes Körpergewebe Abmessungen aufweist, die deutlich geringer sind als diejenigen der Elektroden 32 ,33 ,36 und 37. Es erfolgt eine Abstützung der Maulteilen 18 und 20 relativ zueinander sowohl distal als auch proximal und ferner zu beiden Seiten des Kanals 42 mittels der Abstandsglieder 53 und 54. Beim Überführen der Maulteile 18 und 20 in die Greifstellung lassen sich dadurch ferner Verformungen und Verspannungen der Elektroden 32, 33, 36 und 37 sowie von deren Trägerelementen 34 und 38 weitgehend vermeiden.

Außerdem ist es von Vorteil, dass insgesamt nur zwei Abstandsglieder 53 und 54 zum Einsatz kommen, so dass die Versiegelungsflächen der Elektroden 32, 33, 36 und 37 weitgehend erhalten bleiben. Im Bereich der lediglich punktförmigen Abstandsglieder 53 und 54 kann sich die beim Stromfluss entstehende Wärme ausbreiten, so dass auch Gewebebereiche, die die isolierenden Abstandsglieder 53 und 54 kontaktieren, noch versiegelt werden können.

Anstelle der hier dargestellten punktförmigen Abstandsglieder 53 und 54 können auch Abstandsglieder anderer Form vorgesehen sein, beispielsweise streifenförmige, rechteckförmige oder dreieckförmige Abstandsglieder. Das Albstandsglied 53 könnte auch in eine der Elektroden 32, 36 und 37 integriert sein, und das Abstandsglied 54 in eine der Elektroden 32, 33 und 37.

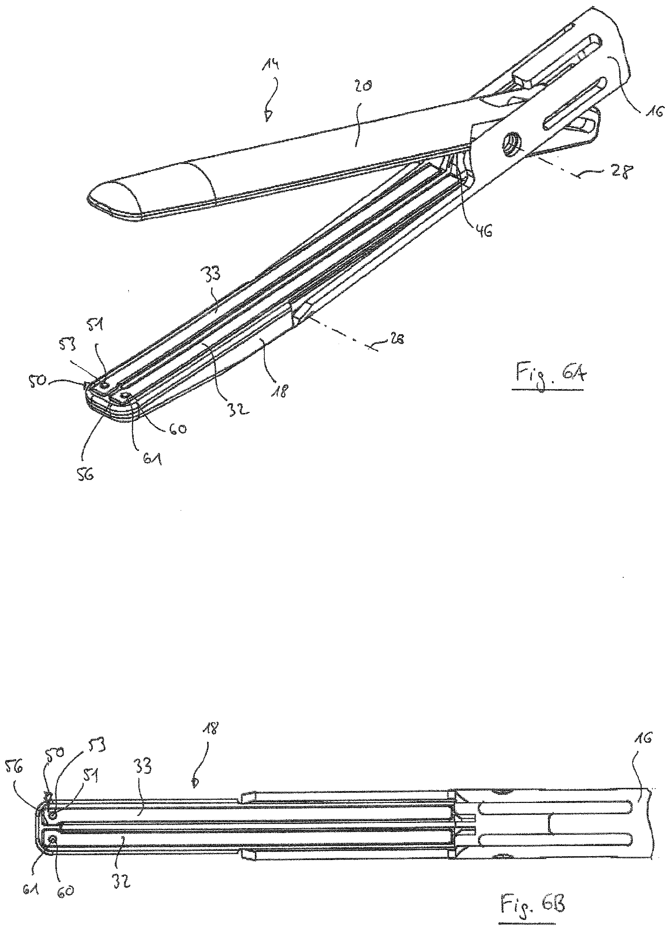

Nachfolgend wird auf eine in den Figuren 6A bis 8B nur abschnittsweise dargestellte bevorzugte zweite Ausführungsform des erfindungsgemäßen Instrumentes Bezug genommen, welches selbst nicht in der Zeichnung dargestellt ist, abgesehen von den nachfolgend beschriebenen Unterschieden aber wie das Instrument 10 ausgestaltet ist. Dies gilt in entsprechender Weise auch für die nachfolgend noch erläuterten weiteren vorteilhaften Ausführungsformen des erfindungsgemäßen Instrumentes.

Die vorstehend erläuterten, mit dem Instrument 10 erzielbaren Vorteile können mit den nun erläuterten weiteren Ausführungsformen ebenfalls erzielt werden. Für gleiche und gleichwirkende Bauteile und Merkmale werden identische Bezugszeichen benutzt.

Bei der zweiten Ausführungsform der Erfindung gemäß den Figuren 6A bis 8B umfasst die Einrichtung 50 anstelle des Abstandsglieds 54 ein Begrenzungselement 60 in Gestalt eines Abstandsglieds 61. Das Abstandsglied 61 ist in die Elektrode 32 integriert, und zwar seitlich neben dem Abstandsglied 53, bezogen auf eine Richtung quer zur Längsrichtung des Instrumentes 10. Damit weist das Maulteil 18 in der zweiten Ausführungsform zwei Abstandsglieder 53 und 61 am distalen Endbereich 56 auf. Beim Überführen der Maulteile 18 und 20 kann ein Kurzschluss zwischen den Elektroden 32 und 36 sowie 33 und 37 am distalen Endbereich 56 besonders wirkungsvoll verhindert werden.

Anders als in den Figuren 6A und 6B dargestellt, könnten die Abstandsglieder 53 und 61 auch an den Elektroden 36 und 37 angeordnet sein.

Zur Begrenzung der Schließweite proximalseitig umfasst die Einrichtung 50 bei der zweiten Ausführungsform zwei weitere Begrenzungselemente 62 und 63 in Gestalt von Anschlagsgliedern 64 bzw. 65. Die Anschlagsglieder 64 und 65 sind etwas proximalseitig der Schwenkachse 26 auf der dem Maulteil 20 abgewandten Seite des Maulteils 18 und des Schaftes 16 angeordnet und jeweils als aneinander anlegbare Absätze ausgestaltet. Das Anschlagsglied 64 ist einstückig mit der Fassung 35 des Maulteils 18 gebildet, und das Anschlagsglied 65 ist einstückig mit dem distalen Ende des Schaftes 16 gebildet.

Nehmen die Maulteile 18 und 20 die Öffnungsstellung ein, sind die Anschlagsglieder 64 und 65 voneinander beabstandet (Figuren 7A und 7B). Beim Überführen der Maulteile 18 und 20 in die Greifstellung kontaktieren die Abstandsglieder 53 und 61 zunächst die Elektroden 37 bzw. 36, da das Maulteil 18 distal in Bezug auf das Maulteil 20 vorgespannt ist. Dies führt zum Verschwenken des Maulteils 18 um die Schwenkachse 26, wobei das proximale Ende des Maulteils 18 auf das proximale Ende des Maulteils 20 zu verschwenkt wird. Die Schwenkbewegung wird durch die Anschlagsglieder 64 und 65 begrenzt, die aneinander zur Anlage geraten (Figur 8B). Dies führt dazu, dass die Schließweite der Maulteilen 18 und 20 proximalseitig durch die Anschlagsglieder 64 und 65 begrenzt wird. Bei der zweiten Ausführungsform gemäß den Figuren 6A bis 8B braucht daher kein proximales Abstandsglied vorgesehen zu sein, welches natürlich trotzdem vorhanden sein könnte,

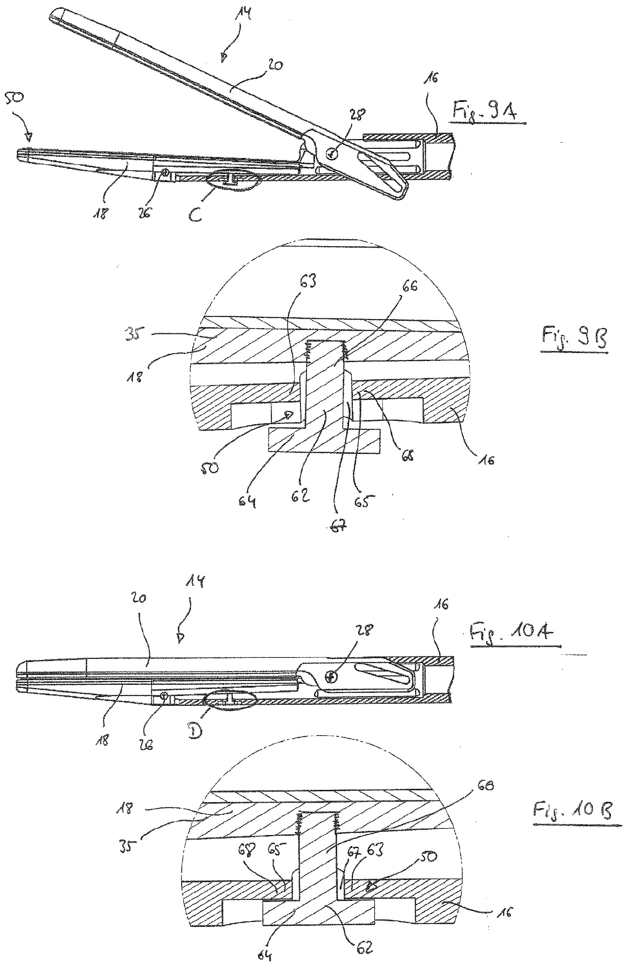

Bei einer in den Figuren 9A bis 10B abschnittsweise gezeigten dritten Ausführungsform der Erfindung sind ebenfalls die Begrenzungselemente 62 und 63 der Einrichtung 50 in Gestalt der Anschlagsglieder 64 und 65 vorgesehen. Das Anschlagsglied 64 ist bei der dritten Ausführungsform ausgestaltet als am Trägerelement 34 festlegbar verstellbares Stellelement in Gestalt einer Schraube 66. Die Schraube 66 durchgreift eine Durchbrechung 67 am distalen Ende des Schaftes 16, deren Rand 68 das Anschlagsglied 65 ausbildet.

Mittels der Schraube 66 kann herstellerseitig oder anwenderseitig die Schließweite der Maulteile 18 und 20 eingestellt werden, je nachdem, wie weit die Schraube 66 in das Trägerelement 34 eingeschraubt wird.

In der Öffnungsstellung der Maulteile 18 und 20 weist ein Kopf der Schraube 66 einen Abstand vom Rand 68 auf. Die Verschwenkung des Maulteils 18 um die Schwenkachse 26, und damit die Schließseite der Maulteilen 18 und 20 proximalseitig wird dadurch begrenzt, dass der Kopf der Schraube 66 beim Überführen der Maulteilen 18 und 20 in die Greifstellung am Rand 68 anschlägt (Figur 10B).

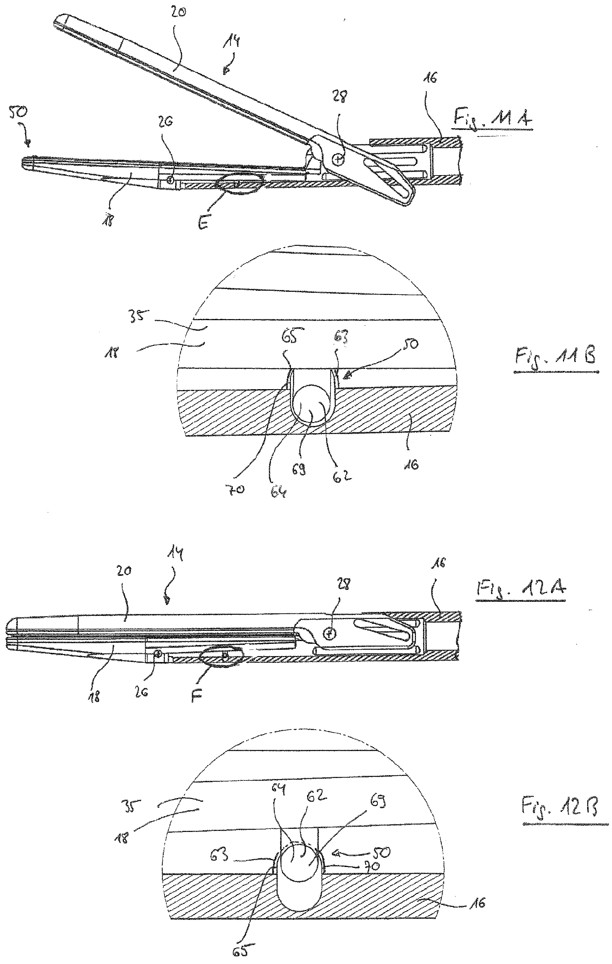

Bei einer in den Figuren 11A bis 12B teilweise dargestellten vierten Ausführungsform der Erfindung sind ebenfalls die Begrenzungselemente 62 und 63 in Gestalt der Anschlagsglieder 64 und 65 vorgesehen. Das Anschlagsglied 64 ist ausgestaltet als am Trägerelement 34 festgelegter Zapfen 69. Der Zapfen 69 greift in das Anschlagsglied 65 in Gestalt eines am distalen Ende des Schaftes 16 festgelegten Langloches 70 ein.

Werden die Maulteile 18 und 20 der vierten Ausführungsform von der Öffnungsstellung (Figuren 11A und 11B) in die Greifstellung überführt, kann sich der Zapfen 69 50 lange im Langloch 70 bewegen, bis er an diesem endseitig anschlägt (Figur 12B). Dies begrenzt die Schwenkbewegung des Maulteils 18 um die Schwenkachse 26 und damit die Schließweite der Maulteile 18 und 20 proximalseitig. Außerdem bilden der Zapfen 69 und das Langloch 70 Führungsglieder zum Führen der Verschwenkung des Maulteils 18 um die Schwenkachse 26.

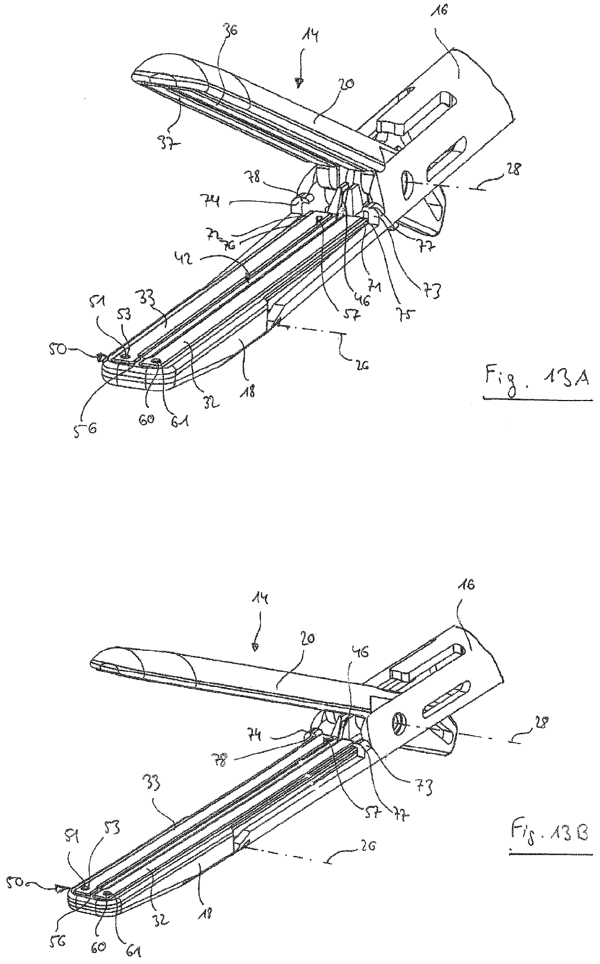

Bei einer in den Figuren 13A und 13B teilweise dargestellten fünften Ausführungsform der Erfindung sind Begrenzungselemente 71 bis 74 der Einrichtung 50 vorgesehen, die jeweils auf der dem Maulteil 20 zugewandten Seite des Maulteils 18 und des Schafts 16 angeordnet sind. Die Begrenzungselemente 71 bis 74 sind jeweils Anschlagsglieder 75 bis 78, wovon die Anschlagsglieder 75 und 76 durch das proximale Ende des Trägerelementes 34 ausgebildet werden. Die Anschlagsglieder 75 und 76 sind an einem Außenrand seitlich neben den Elektroden 32 bzw. 33 angeordnet.

Die Anschlagsglieder 77 und 78 sind am Schaft 16 etwas distal der Schwenkachse 28 angeordnet, jeweils hakenförmig ausgestaltet, und sie übergreifen das Trägerelement 34. Nehmen die Maulteile 18 und 20 der fünften Ausführungsform die Öffnungsstellung ein, sind die Anschlagsglieder 75 und 77 ebenso voneinander beabstandet wie die Anschlagsglieder 76 und 78 (Figur 13A). Werden die Maulteile 18 und 20 in die Greifstellung überführt, wird dadurch das Maulteil 18 um die Schwenkachse 26 verschwenkt. Dabei können die Anschlagsglieder 75 und 77 sowie 76 und 78 aneinander zur Anlage geraten, um die Verschwenkung des Maulteils, 18 zu begrenzen. Dies ist in Figur 13B dargestellt, wobei die Maulteile 18 und 20 nur zur besseren Sichtbarkeit nicht die Greifstellung einnehmen, sondern die Öffnungsstellung. Damit dienen die Anschlagsglieder 75 bis 78 zur proximalseitigen Begrenzung der Schließweite der Maulteile 18 und 20.

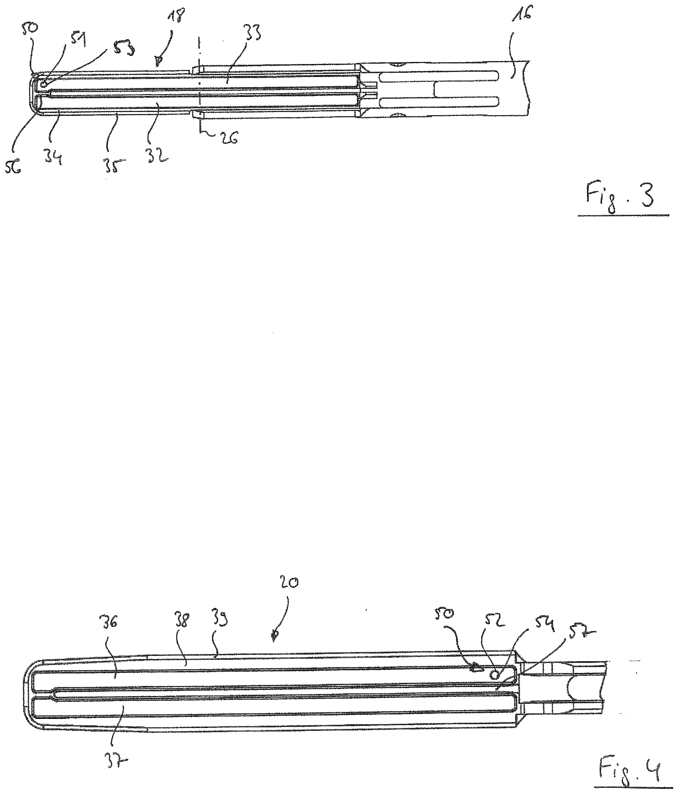

Begrenzungselemente und Anschlagsglieder, wie sie vorstehend unter Verweis auf die Figuren 7A bis 13B beschrieben wurden, könnten auch bei der ersten Ausführungsform der Erfindung gemäß den Figuren 1 bis 5 vorgesehen sein.

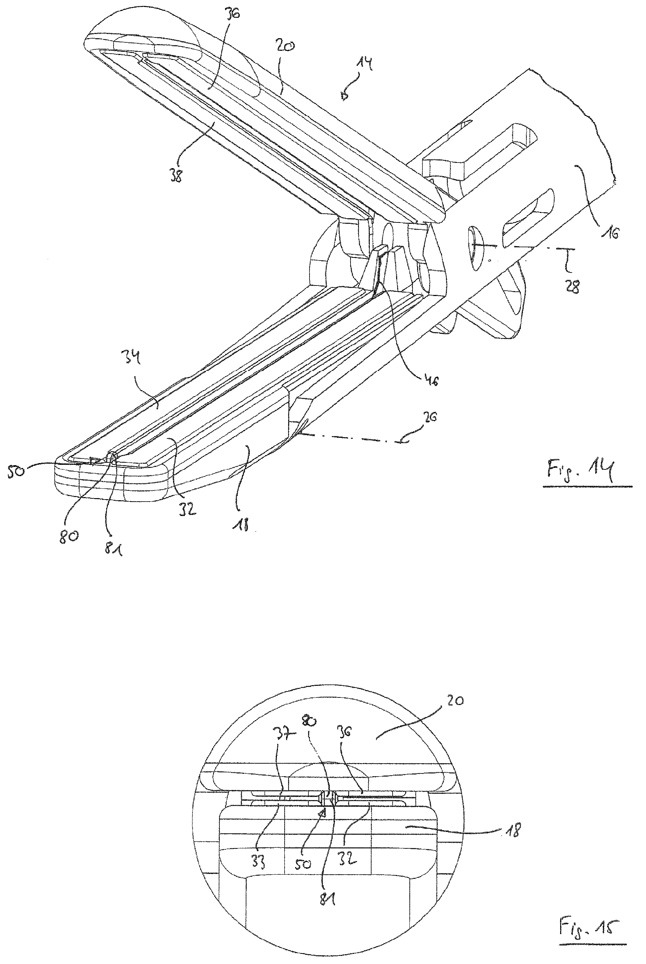

Des Weiteren sind Begrenzungselemente und Anschlagsglieder, wie sie unter Verweis auf die Figuren 7A bis 13B beschrieben wurden, bei der nachfolgend erläuterten und in den Figuren 14 und 15 teilweise dargestellten sechsten Ausführungsform der Erfindung vorhanden zur dortigen Begrenzung der Schließweite der Maulteile 18 und 20.

Bei der sechsten Ausführungsform umfasst die Einrichtung 50 am distalen Endbereich 56 nur ein einziges Begrenzungselement 80 in Gestalt eines Abstandsgliedes 81. Das Abstandsglied 81 ist distalseitig des Kanals 42 zwischen den distalen Enden der Elektroden 32 und 33 angeordnet und einstückig mit dem Trägerelement 34 gebildet. Demgegenüber ist bei der sechsten Ausführungsform kein Abstandsglied in eine der Elektroden 32, 33, 36 und 37 integriert. Dies könnte selbstverständlich ebenfalls vorgesehen sein.

Das Abstandsglied 81 ist so bemessen, dass die Elektroden 32 und 36 sowie 33 und 37 in der Greifstellung der Maulteile 18 und 20 (Figur 15) noch einen Abstand voneinander aufweisen, der ungefähr gleich dem durch die Abstandsglieder 53 und 54 erzielbaren Abstand ist.

Über das Abstandsglied 81 hinaus könnten am Maulteil 18 oder am Maulteil 20 weitere Abstandsglieder vorgesehen sein, die einstückig mit dem Trägerelement 34 bzw. dem Trägerelement 38 gebildet sind.

Anstelle der in den Figuren 14 und 15 dargestellten nippelförmigen Gestalt könnte das Abstandsglied 81 auch eine andersartige Gestalt aufweisen und beispielsweise zylindrisch, rippenförmig, kegel- oder kegelstumpfförmig, pyramidal oder pyramidenstumpfförmig sein. Das Abstandsglied 81 könnte auch distal der Elektroden 32 und 33 angeordnet sein.

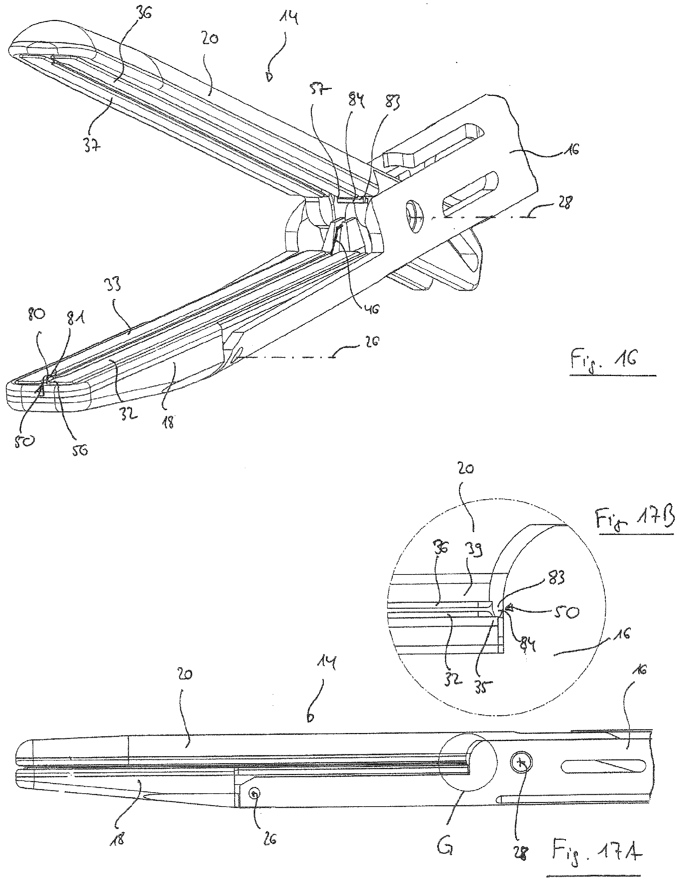

Eine in den Figuren 16 bis 17A dargestellte siebte Ausführungsform der Erfindung unterscheidet sich von der vorstehend erläuterten sechsten Ausführungsform gemäß den Figuren 14 und 15 dadurch, dass die Schließseite der Maulteile 18 und 20 bei der siebten Ausführungsform proximalseitig mittels eines Begrenzungselementes 83 der Einrichtung 50 begrenzbar ist. Das Begrenzungselement 83 ist ausgestaltet als Abstandsglied 84, das proximalseitig der Elektrode 36 angeordnet und einstückig mit dem Trägerelement 38 gebildet ist. Das Abstandsglied 84 ist leistenförmig ausgestaltet, und es steht vom Trägerelement 38 50 weit in Richtung des Maulteils 18 ab wie das Abstandsglied 81 vom Trägerelement 34 in Richtung des Maulteils 20.

Nehmen die Maulteile 18 und 20 der siebten Ausführungsform die Öffnungsstellung ein, ist das Abstandsglied 84 relativ zum Trägerelement 34 beabstandet (Figur 16). Nehmen die Maulteile 18 und 20 die Greifstellung ein, kann das Abstandsglied 84 am proximalen Ende des Trägerelements 24 anliegen und damit die Schließweite der Maulteile 18 und 20 proximalseitig begrenzen (Figuren 17A und 17B).

In entsprechender Weise könnte proximalseitig der Elektrode 33 oder der Elektrode 37 ein weiteres proximales Abstandsglied bei der siebten Ausführungsform vorhanden sein. Ebenso könnte das Abstandsglied 84 bei den weiteren, hier erläuterten Ausführungsformen der Erfindung vorhanden sein.

Vorstehende Beschreibung zeigt, dass sämtliche Ausführungsformen der Erfindung zumindest ein am distalen Endbereich 56 der Maulteilanordnung 14 angeordnetes Abstandsglied umfassen zum distalseitigen Begrenzen der Schließweite der Maulteile 18 und 20. Zudem sind maximal zwei Abstandsglieder pro Maulteil 18 oder 20 vorhanden. In der Greifstellung können sich die Maulteile 18 und 20 daher zur Vermeidung von elektrischen Kurzschlüssen aneinander abstützen. Zugleich kann ein gutes Versiegelungsergebnis erzielt werden, da die Versiegelungswirkung der Elektroden 32, 33, 36 und 37 durch die geringen Abmessungen und die geringe Anzahl der Abstandsglieder nicht beeinträchtigt wird.

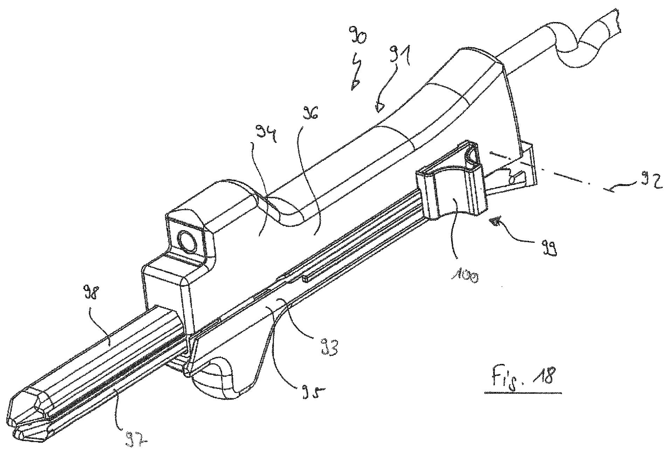

Figur 18 zeigt in perspektivischer Darstellung eine achte bevorzugte Ausführungsform eines erfindungsgemäßen Instrumentes, die mit dem Bezugszeichen 90 belegt ist. Das Instrument 90 ist ebenfalls ein bipolares elektrochirurgisches Instrument, und die mit dem Instrument 10 erzielbaren Vorteile können auch mit dem Instrument 90 erzielt werden. Diesbezüglich wird auf vorstehende Ausführungen verwiesen.

Das Instrument 90 weist eine Handhabe 91 auf, die zwei relativ zueinander um eine Schwenkachse 92 verschwenkbare Griffelemente 93 und 94 umfasst. Jedes der Griffelemente 93, 94 bildet einen Maulteilträger 95 bzw. 96. Am Maulteilträger 95 ist distal ein Maulteil 97 festgelegt, und am Maulteil 96 ist distal ein Maulteil 98 festgelegt.

Die Maulteile 97 und 98 können alle diejenigen Merkmale und Bauteile aufweisen, die vorstehend am Beispiel der Maulteilen 18 bzw. 20 erläutert wurden. Beispielsweise kann das Maulteil 97 dem Maulteil 18 entsprechen und das Maulteil 98 dem Maulteil 20, oder umgekehrt. Entsprechendes gilt für die Maulteilträger 95 und 96 in Bezug auf den Maulteilträger 25. Werden die Griffelemente 93 und 94 um die Schwenkachse 92 relativ zueinander verschwenkt, können die Maulteile 97 und 98 von einer Öffnungsstellung in eine Greifstellung überführt werden, um zu versiegelndes Körpergewebe zu greifen.

Auch das Instrument 90 weist eine Schneideinrichtung 99 auf mit einem an der Handhabe 91 angeordneten Betätigungsglied 100. Ein in der Zeichnung nicht dargestelltes Schneidelement kann dadurch in proximal-distaler Richtung verschoben werden, um zwischen den Maulteilen 97 und 98 gefasstes Körpergewebe zu durchtrennen.

## Patentansprüche

1. Elektrochirurgisches Instrument (10) mit einem proximalen Ende und einem distalen Ende, umfassend:
- eine Maulteilanordnung (14) am distalen Ende, die ein erstes Maulteil (18) sowie ein relativ zu diesem bewegliches zweites Maulteil (20) aufweist, wobei die Maulteile (18, 20) relativ zueinander von einer Öffnungsstellung in eine Greifstellung überführbar sind, in welcher Greifstellung sie näher aneinander angeordnet sind als in der Öffnungsstellung, wobei jedes Maulteil (18, 20) mindestens eine mit einer elektrischen Energiequelle verbindbare Elektrode (32, 33, 36, 37) aufweist und Elektroden (32, 33, 36, 37) beider Maulteile (18, 20) zum Versiegeln von in der Greifstellung zwischen den Maulteilen (18, 20) gehaltenem Körpergewebe zusammenwirken können;
- eine Betätigungseinrichtung (12) zum Überführen der Maulteile (18, 20) von der Öffnungsstellung in die Greifstellung, und
- eine Einrichtung (50) zum Begrenzen der Schließweite der Maulteile (18, 20), wenn diese von der Öffnungsstellung in die Greifstellung überführt werden,
wobei
die Einrichtung (50) eine Mehrzahl von Begrenzungselementen (51, 52) aufweist, **dadurch gekennzeichnet, dass** insgesamt nur zweig der Begrenzungselemente als ein vom Maulteil (18, 20) in Richtung des anderen Maulteils (20, 18) über eine von der mindestens einen Elektrode (32, 33, 36, 37) des Maulteils 18, 20) ausgebildete Versiegelungsfläche hervorspringendes, elektrisch isolierendes Abstandsglied (53, 54) ausgestaltet sind, wobei die zwei Abstandsglieder (53, 54) an verschiedenen Maulteilen (18, 20) angeordnet sind und mindestens ein Abstandsglied (53, 54) an einem distalen Endbereich der Maulteilanordnung (14) angeordnet ist.

2. Elektrochirurgisches Instrument (10) nach Anspruch 1, **dadurch gekennzeichnet, dass** zumindest ein Abstandsglied (54) proximal bezüglich des distalen Endbereiches, insbesondere an einem proximalen Endbereich des Maulteils (20) angeordnet ist.

3. Elektrochirurgisches Instrument (10) nach einem der voranstehenden Ansprüche, **dadurch gekennzeichnet, dass** mindestens ein Abstandsglied (53, 54), insbesondere alle Abstandsglieder, einstückig mit einem Trägerelement (34, 38) desjenigen Maulteils (18, 20), an dem es angeordnet ist, für die mindestens eine Elektrode (32, 33, 36, 37) dieses Maulteils (18, 20) gebildet ist und die Elektrode (32, 33, 36, 37) durchgreift.

4. Elektrochirurgisches Instrument (10) nach einem der voranstehenden Ansprüche, **dadurch gekennzeichnet, dass** das Instrument eine Schneideinrichtung (44) umfasst zum Durchtrennen von zwischen den Maulteilen (18, 20) in deren Greifstellung gehaltenem Körpergewebe, wobei die Schneideinrichtung (44) ein Schneidelement (46) aufweist, das quer zur proximal-distalen Richtung zwischen zwei Versiegelungsflächenabschnitten der jeweiligen mindestens einen Elektrode (32, 33, 36, 37) der Maulteile (18, 20) angeordnet oder anordenbar ist.

5. Elektrochirurgisches Instrument (10) nach Anspruch 4, **dadurch gekennzeichnet, dass** die Schneideinrichtung (44) ein Betätigungselement (45) zum Bewegen des Schneidelementes (46) in proximal-distaler Richtung und zu dessen Überführen von einer zurückgezogenen Stellung in eine vorgeschobene Stellung umfasst, wobei beim Überführen zwischen den Maulteilen (18, 20) in deren Greifstellung gehaltenes Körpergewebe durchtrennt wird.

6. Elektrochirurgisches Instrument (10) nach Anspruch 4 oder 5, **dadurch gekennzeichnet, dass** zumindest ein Maulteil (18, 20) zwei getrennte Elektroden (32, 33, 36, 37) aufweist, von denen jede einen Versiegelungsflächenabschnitt ausbildet.

7. Elektrochirurgisches Instrument (10) nach einem der Ansprüche 4 bis 6, **dadurch gekennzeichnet, dass** quer zur proximal-distalen Richtung genau ein Abstandsglied (53, 54) zu jeder Seite des Schneidelementes (46) angeordnet ist.

8. Elektrochirurgisches Instrument (10) nach einem der voranstehenden Ansprüche, **dadurch gekennzeichnet, dass** die Mehrzahl von Begrenzungselementen (51, 52) mindestens ein weiteres, nicht als Abstandsglied ausgestaltetes Begrenzungselement (64, 65) umfasst, das proximal bezüglich des mindestens einen, am distalen Endbereich der Maulteilanordnung (14) angeordneten Abstandsgliedes (53, 54) angeordnet ist.

9. Elektrochirurgisches Instrument (10) nach einem der voranstehenden Ansprüche, **dadurch gekennzeichnet, dass** das Instrument einen Maulteilträger (25) für das erste Maulteil (18) aufweist, relativ zu dem das zweite Maulteil (20) verschwenkbar ist zum Überführen der Maulteile (18, 20) von der Öffnungsstellung in die Greifstellung, und dass das erste Maulteil (18) relativ zum Maulteilträger (25) um eine quer zu einer von diesem definierten Längsrichtung ausgerichtete Schwenkachse schwenkbar ist.

10. Elektrochirurgisches Instrument (10) nach Anspruch 9, **dadurch gekennzeichnet, dass** die Mehrzahl von Begrenzungselementen (51, 52) mindestens ein weiteres, als erstes Anschlagsglied (64) ausgestaltetes Begrenzungselement umfasst, das am ersten Maulteil (18) angeordnet ist, sowie mindestens ein weiteres, als zweites Anschlagsglied (65) ausgestaltetes Begrenzungselement, das am Maulteilträger (25) angeordnet ist und das zum Begrenzen der Schwenkbewegung des ersten Maulteils (18) relativ zum Maulteilträger (25) derart, dass das proximale Ende des ersten Maulteils (18) hin zum zweiten Maulteil (20) verschwenkt wird, zusammenwirkt.

11. Elektrochirurgisches Instrument (10) nach Anspruch 10, **dadurch gekennzeichnet, dass** die Anschlagsglieder (64, 65) die Schwenkbewegung derart begrenzen, dass bei deren Zusammenwirken die von der mindestens einen Elektrode (32, 33) des ersten Maulteils (18) ausgebildete Versiegelungsfläche parallel zur vom Maulteilträger (25) definierten Längsrichtung ausgerichtet ist.

12. Elektrochirurgisches Instrument (10) nach Anspruch 10 oder 11, **dadurch gekennzeichnet, dass** die Anschlagsglieder (64, 65) als aneinander anlegbare Absätze des ersten Maulteils (18) bzw. des Maulteilträgers (25) ausgebildet sind.

13. Elektrochirurgisches Instrument (10) nach einem der Ansprüche10 bis 12, **dadurch gekennzeichnet, dass** die Anschlagsglieder (64, 65) Führungsglieder (69, 70) zum Führen des ersten Maulteils (18) beim Verschwenken relativ zum Maulteilträger (25) ausbilden.

14. Elektrochirurgisches Instrument (10) nach einem der Ansprüche 10 bis 13, **dadurch gekennzeichnet, dass** ein Anschlagsglied (65) als Langloch (70) ausgebildet ist, das mit ihm zusammenwirkende Anschlagsglied (64) als in das Langloch (70) eingreifender Zapfen (69).

15. Elektrochirurgisches Instrument (10) nach einem der Ansprüche10 bis 14, **dadurch gekennzeichnet, dass** mindestens ein Anschlagsglied (64) ein verstellbares Stellglied (66) ausbildet, das relativ zum ersten Maulteil (18) bzw. relativ zum Maulteilträger (25) verstellbar ist.

## Claims

1. An electrosurgical instrument (10) having a proximal end and a distal end comprising:
- a jaw member assembly (14) at the distal end including a first jaw member (18) and a second jaw member (20) movable relative thereto, wherein the jaw members (18, 20) can be transferred relative to each other from an opening position into a gripping position in which gripping position they are arranged to be closer to each other than in the opening position, each jaw member (18, 20) having at least one electrode (32, 33, 36, 37) connectable to an electric power source and electrodes (32, 33, 36, 37) of both jaw members (18, 20) being adapted to interact for sealing body tissue held between the jaw members (18, 20) in the gripping position;
- actuating means (12) for transferring the jaw members (18, 20) from the opening position into the gripping position; and
- means (50) for limiting the closing width of the jaw members (18, 20) when they are transferred from the opening position into the gripping position,
wherein the means (50) includes a plurality of limiting elements (51, 52), **characterized in that** a total of only two of the limiting elements (51, 52) are in the form of an electrically insulating spacer (53, 54) projecting from the jaw member (18, 20) in the direction of the other jaw member (20, 18) over a sealing surface formed by the at least one electrode (32, 33, 36, 37) of the jaw member, wherein the two spacers (53, 54) are arranged at different jaw members (18, 20) and at least one spacer (53, 54) thereof is arranged at a distal end portion of the jaw member assembly (14).

2. The electrosurgical instrument (10) according to claim 1, **characterized in that** at least one spacer (54) is arranged proximally relative to the distal end portion, especially at a proximal end portion of the jaw member (20).

3. The electrosurgical instrument according to any one of the preceding claims, **characterized in that** at least one spacer (53, 54), in particular all spacers, is formed integrally with a support element (34, 38) of the jaw member (18, 20) at which it is disposed for the at least one electrode (32, 33, 36, 37) of said jaw member (18, 20) and passes through the electrode (32, 33, 36, 37).

4. The electrosurgical instrument (10) according to any one of the preceding claims, **characterized in that** the instrument comprises a cutting means (44) for severing body tissue held between the jaw members (18, 20) in the gripping position thereof, wherein the cutting means (44) includes a cutting element (46) that is or can be arranged transversely to the proximal-distal direction between two sealing surface portions of the respective at least one electrode (32, 33, 36, 37) of the jaw members (18, 20).

5. The electrosurgical instrument (10) according to claim 4, **characterized in that** the cutting means (44) comprises an actuating element (45) for moving the cutting element (46) in the proximal-distal direction and for transferring the latter from a retracted position into an advanced position, wherein during transfer body tissue held between the jaw members (18, 20) in the gripping position thereof is severed.

6. The electrosurgical instrument (10) according to any one of the claims 4 or 5, **characterized in that** at least one jaw member (18, 20) includes two separate electrodes (32, 33, 36, 37) each of which forms a sealing surface portion.

7. The electrosurgical instrument (10) according to any one of the claims 4 to 6, **characterized in that** transversely to the proximal-distal direction exactly one spacer (53, 54) is arranged on each side of the cutting element (46).

8. The electrosurgical instrument (10) according to any one of the preceding claims, **characterized in that** the plurality of limiting elements (51, 52) comprises at least one further limiting element (64, 65) not configured as a spacer which is arranged proximally with respect to the at least one spacer (53, 54) disposed at the distal end portion of the jaw member assembly (14).

9. The electrosurgical instrument (10) according to any one of the preceding claims, **characterized in that** the instrument includes a jaw member support (25) for the first jaw member (18) relative to which the second jaw member (20) can be pivoted for transferring the jaw members (18, 20) from the opening position into the gripping position, and **in that** the first jaw member (18) can be pivoted relative to the jaw member support (25) about a pivot axis orientated transversely to a longitudinal direction defined by the jaw member support (25).

10. The electrosurgical instrument (10) according to claim 9, **characterized in that** the plurality of limiting elements (51, 52) comprises at least one further limiting element configured as a first stop member (64) that is disposed at the first jaw member (18) as well as at least one further limiting element configured as a second stop member (65) that is disposed at the jaw member support (25) and interacts for limiting the pivoting motion of the first jaw member (18) relative to the jaw member support (25) such that the proximal end of the first jaw member (18) is pivoted toward the second jaw member (20).

11. The electrosurgical instrument (10) according to claim 10, **characterized in that** the stop members (64, 65) limit the pivoting motion so that upon interaction thereof the sealing surface formed by the at least one electrode (32, 33) of the first jaw member (18) is orientated in parallel to the longitudinal direction defined by the jaw member support (25).

12. The electrosurgical instrument (10) according to claim 10 or 11, **characterized in that** the stop members (64, 65) are in the form of steps of the first jaw member (18) and of the first jaw member (18) support, respectively, adapted to be attached to each other.

13. The electrosurgical instrument (10) according to any one of the claims 10 to 12, **characterized in that** the stop members (64, 65) constitute guide members (69, 70) for guiding the first jaw member (18) during pivoting relative to the jaw member support (25).

14. The electrosurgical instrument (10) according to any one of the claims 10 to 13, **characterized in that** a stop member (65) is formed as oblong hole (70) and the stop member (64) interacting therewith is formed as pin (69) engaging in said oblong hole (70).

15. The electrosurgical instrument (10) according to any one of the claims 10 to 14, **characterized in that** at least one stop member (64) forms an adjustable actuator (66) that is adjustable relative to the first jaw member (18) and relative to the jaw member support (25), respectively.

## Revendications

1. Instrument électrochirurgical (10) pourvu d'une extrémité proximale et d'une extrémité distale, comprenant :
- un ensemble d'éléments de mâchoire (14) au niveau de l'extrémité distale, qui présente un premier élément de mâchoire (18) ainsi qu'un deuxième élément de mâchoire (20) mobile par rapport au premier élément de mâchoire, sachant que les éléments de mâchoire (18, 20) peuvent être transférés l'un par rapport à l'autre depuis une position d'ouverture dans une position de préhension, dans laquelle position de préhension ils sont disposés davantage à proximité l'un de l'autre que dans la position d'ouverture, sachant que chaque élément de mâchoire (18, 20) présente au moins une électrode (32, 33, 36, 37) pouvant être reliée à une source d'énergie électrique et que des électrodes (32, 33, 36, 37) des deux éléments de mâchoire (18, 20) peuvent coopérer afin de sceller un tissu corporel maintenu, dans la position de préhension, entre les éléments de mâchoire (18, 20) ;
- un dispositif d'actionnement (12) servant à transférer les éléments de mâchoire (18, 20) de la position d'ouverture dans la position de préhension ; et
- un dispositif (50) servant à limiter le degré d'ouverture minimal entre les éléments de mâchoire (18, 20) lorsque ces derniers sont transférés depuis la position d'ouverture dans la position de préhension,
sachant que le dispositif (50) présente une multitude d'éléments de limitation (51, 52), **caractérisé en ce qu'**au total seulement deux éléments de limitation sont configurés sous la forme d'un organe d'écartement (53, 54) dépassant depuis l'élément de mâchoire (18, 20) en direction de l'autre élément de mâchoire (20, 18) au-delà d'une surface de scellement réalisée par au moins l'électrode (32, 33, 36, 37) au moins au nombre de une de l'élément de mâchoire (18, 20), électriquement isolant, sachant que les deux organes d'écartement (53, 54) sont disposés au niveau de différents éléments de mâchoire (18, 20) et qu'au moins un organe d'écartement (53, 54) est disposé au niveau d'une zone d'extrémité distale de l'ensemble d'éléments de mâchoire (14).

2. Instrument électrochirurgical (10) selon la revendication 1, **caractérisé en ce qu'**au moins un organe d'écartement (54) est disposé de manière proximale par rapport à la zone d'extrémité distale, en particulier au niveau d'une zone d'extrémité proximale de l'élément de mâchoire (20).

3. Instrument électrochirurgical (10) selon l'une quelconque des revendications précédentes, **caractérisé en ce qu'**au moins un organe d'écartement (53, 54), en particulier tous les organes d'écartement, est formé d'un seul tenant avec un élément de support (34, 38) de l'élément de mâchoire (18, 20) précisément au niveau duquel l'organe d'écartement est disposé, pour l'électrode (32, 33, 36, 37) au moins au nombre de une dudit élément de mâchoire (18, 20) et qui traverse l'électrode (32, 33, 36, 37).

4. Instrument électrochirurgical (10) selon l'une quelconque des revendications précédentes, **caractérisé en ce que** l'instrument comprend un dispositif tranchant (44) servant à découper un tissu corporel maintenu entre les éléments de mâchoire (18, 20) dans la position de préhension de ces derniers, sachant que le dispositif tranchant (44) présente un élément tranchant (46), qui est disposé ou peut être disposé de manière transversale par rapport à la direction proximale-distale entre deux sections de surface de scellement de l'électrode (32, 33, 36, 37) respective au moins au nombre de une des éléments de mâchoire (18, 20).

5. Instrument électrochirurgical (10) selon la revendication 4, **caractérisé en ce que** le dispositif tranchant (44) comprend un élément d'actionnement (45) servant à déplacer l'élément tranchant (46) dans la direction proximale-distale et servant à le transférer d'une position en retrait dans une position en avant, sachant qu'un tissu corporel maintenu entre les éléments de mâchoire (18, 20) dans la position de préhension de ces derniers est découpé lors du transfert.

6. Instrument électrochirurgical (10) selon la revendication 4 ou 5, **caractérisé en ce qu'**au moins un élément de mâchoire (18, 20) présente deux électrodes (32, 33, 36, 37) séparées, dont chacune forme une section de surface de scellement.

7. Instrument électrochirurgical (10) selon l'une quelconque des revendications 4 à 6, **caractérisé en ce que** précisément un organe d'écartement (53, 54) par rapport à chaque côté de l'élément tranchant (46) est disposé de manière transversale par rapport à la direction proximale-distale.

8. Instrument électrochirurgical (10) selon l'une quelconque des revendications précédentes, **caractérisé en ce que** la multitude d'éléments de limitation (51, 52) comprend au moins un autre élément de limitation (64, 65) non configuré sous la forme d'un organe d'écartement, lequel est disposé de manière proximale par rapport à l'organe d'écartement (53, 54) au moins au nombre de un disposé au niveau de la zone d'extrémité distale de l'ensemble d'éléments de mâchoire (14).

9. Instrument électrochirurgical (10) selon l'une quelconque des revendications précédentes, **caractérisé en ce que** l'instrument présent un support d'élément de mâchoire (25) pour le premier élément de mâchoire (18), par rapport auquel le deuxième élément de mâchoire (20) peut pivoter afin de transférer les éléments de mâchoire (18, 20) de la position d'ouverture dans la position de préhension, et **en ce que** le premier élément de mâchoire (18) peut pivoter, par rapport au support d'élément de mâchoire (25), autour d'un axe de pivotement orienté de manière transversale par rapport à une direction longitudinale définie par ce dernier.

10. Instrument électrochirurgical (10) selon la revendication 9, **caractérisé en ce que** la multitude d'éléments de limitation (51, 52) comprend au moins un autre élément de limitation configuré en tant que premier organe de butée (64), qui est disposé au niveau du premier élément de mâchoire (18), ainsi qu'au moins un autre élément de limitation configuré sous la forme d'un deuxième organe de butée (65), qui est disposé au niveau du support d'élément de mâchoire (25) et qui coopère afin de limiter le déplacement par pivotement du premier élément de mâchoire (18) par rapport au support d'élément de mâchoire (25) de telle manière que l'extrémité proximale du premier élément de mâchoire (18) est pivotée en direction du deuxième élément de mâchoire (20).

11. Instrument électrochirurgical (10) selon la revendication 10, **caractérisé en ce que** les organes de butée (64, 65) limitent le déplacement par pivotement de telle manière que lors de leur coopération, la surface de scellement réalisée par l'électrode (32, 33) au moins au nombre de une du premier élément de mâchoire (18) est orientée de manière parallèle par rapport à la direction longitudinale définie par le support d'élément de mâchoire (25).

12. Instrument électrochirurgical (10) selon la revendication 10 ou 11, **caractérisé en ce que** les organes de butée (64, 65) sont réalisés sous la forme de gradins pouvant être placés l'un contre l'autre du premier élément de mâchoire (18) ou du support d'élément de mâchoire (25).

13. Instrument électrochirurgical (10) selon l'une quelconque des revendications 10 à 12, **caractérisé en ce que** les organes de butée (64, 65) réalisent des organes de guidage (69, 70) servant à guider le premier élément de mâchoire (18) lors du pivotement par rapport au support d'élément de mâchoire (25).

14. Instrument életrochirurgical (10) selon l'une quelconque des revendications 10 à 13, **caractérisé en ce qu'**un organe de butée (65) est réalisé sous la forme d'un trou oblong (70), **en ce que** l'organe de butée (64) coopérant avec lui est réalisé sous la forme d'un tenon (69) venant en prise avec le trou oblong (70).

15. Instrument életrochirurgical (10) selon l'une quelconque des revendications 10 à 14, **caractérisé en ce qu'**au moins un organe de butée (64) réalise un organe de réglage (66) ajustable, qui peut être ajusté par rapport au premier élément de mâchoire (18) ou par rapport au support d'élément de mâchoire (25).
